# FASCICULE DE BREVET EUROPEEN

(11) **EP 0 476 107 B1**
(45) Date de publication et mention de la délivrance du brevet: **19.06.1996**
(21) Numéro de dépôt: 91907280.1
(22) Date de dépôt: 22.03.1991
(51) Int. Cl.: C12Q 1/04, C12Q 1/37

(54) **PROCEDE D'IDENTIFICATION DE CANDIDA ALBICANS AU MOYEN DE SUBSTRATS CHROMOGENES**
VERFAHREN ZUR IDENTIFIZIERUNG VON CANDIDA ALBICANS MITTELS CHROMOGENER SUBSTANZEN
PROCESS FOR IDENTIFYING CANDIDA ALBICANS BY MEANS OF CHROMOGENIC SUBSTANCES

(30) Priorité: 23.03.1990 FR 9003726
(43) Date de publication de la demande: 25.03.1992
(73) Titulaire: DIAGNOSTICA STAGO (société anonyme), F-92602 Asnières (FR)
(72) Inventeur: LEPARGNEUR, Jean-Pierre Route de Rebique, F-31320 Pechabou (FR); PUSSARD-CONTANT, Geneviève La Corderie, F-92400 Courbevoie (FR); MARTINOLI, Jean-Luc, F-92390 Villeneuve-la-Garenne (FR); QUENTIN, Gérard, F-92700 Colombes (FR)
(74) Mandataire: Clisci, Serge
(86) Numéro de dépôt international: FR9100233
(87) Numéro de publication internationale: WO9114787

(56) Documents cités:
- EP-A- 0 173 944
- EP-A- 0 255 341
- US-A- 4 874 695

## Description

### DOMAINE DE L'INVENTION

La présente invention a trait à un nouveau procédé pour la détermination, le dosage et/ou l'identification de souches de Candida albicans parmi les autres souches de ***Candida*** et les souches apparentées appartenant à l'ensemble des ***Torulopsis***, au moyen de substrats chromogènes du type monoaminoacide ou peptidique.

### ART ANTERIEUR

Les candidoses sont provoquées par des souches pathogènes de ***Candida*** ou de souches apparentées appartenant principalement à l'ensemble des ***Torulopsis***. On sait que, dans plus de 60 % des cas rencontrés, les souches de ***Candida*** responsables des candidoses appartiennent à l'espèce ***Candida albicans*** et que, dans les autres cas, les souches responsables appartiennent aux autres espèces de ***Candida***, par exemple ***Candida tropicalis***, ***Candida krusei*** ou à l'ensemble des souches apparentées de ***Torulopsis***, notamment l'espèce ***Torulopsis glabrata***.

On sait que, pour pallier les difficultés (nombre important de manipulations et longues durées d'essais) des techniques antérieures [test de blastèse (ou test de filamentation en sérum), test de chlamydosporulation sur milieux PCB ou RAT] pour l'identification de souches de ***Candida***, principalement les souches de l'espèce ***Candida albicans***, et des souches apparentées, on a proposé des techniques connues dans le domaine biochimique pour détecter des enzymes caractéristiques de levures, telles que les osidases et aminopeptidases, et faisant appel à des substrats chromogènes ou fluorogènes.

Jusqu'à présent, toutes les techniques d'identification de ***Candida*** et de ***Torulopsis*** par détection d'osidases ou d'aminopeptidases ont été réalisées sur des souches préalablement isolées.

On connaît en particulier de D.G. BOBEY et al., J. Clin. Microbiol., 13, (No. 2), pages 393-394, (1981), de J.L. PERRY et al., J. Clin. Microbiol., 25, (No. 12), pages 2424-2425, (1987) et de C.M. SMITKA et al., J. Clin. Microbiol., 27 (No. 1), pages 203-206, (1989), des essais de substrats osidiques marqués par le reste fluorogène 4-méthylumbelliféryle (en abrégé 4MU), à savoir notamment les 4MU-β-D-glucopyranoside, 4MU-2-acétamido-2-désoxy-β-D-glucopyranoside, 4MU-β-D-galactopyranoside et, respectivement, 4Mu-2-acétamido-2-désoxy-β-D-galactopyranoside, en vue de la détection de β-D-glucosidase, N-acétyl-β-D-glucosaminidase, β-D-galactosidase et, respectivement, N-acétyl-β-D-galactosaminidase provenant de souches de ***Candida***. Selon ces documents, il se trouve que les souches de ***Candida*** et de ***Torulopsis*** ne présentent aucune activité β-D-galactosidase, β-L-fucosidase et β-D-glucosidase; par contre les souches de ***Candida albicans*** et quelques souches de ***Candida tropicalis*** présentent une activité N-acétyl-β-D-galactosaminidase. Il est clair que l'utilisation de ces substrats pris isolément n'est pas directement applicable à l'identification de souches de ***Candida albicans*** dans un mélange de souches de ***Candida*** et le cas échéant de ***Torulopsis***.

On connaît également de M.CASAL et al., Mycopathologia 81, pages 155-159, (1983) et de I.POLACHEK et al., J. Clin. Microbiol., 25, pages 907-910, (1987) l'utilisation de substrats osidiques comportant un groupe fluorogène ou chromogène tel que p-nitrophényle (en abrégé : PNP), β-napthyle ou 6-bromo-β-naphtyle [obtenus par couplage (selon la formation d'un pont éther) d'un ose avec le p-nitrophénol, le β-naphtol ou respectivement le 6-bromo-β-naphtol], pour la détermination de souches isolées de ***Candida albicans***. Selon l'enseignement de ces deux documents, lesdits substrats osidiques réagissent avec leur osidase cible provenant des souches de ***Candida*** et des souches apparentées. Toutefois, il convient de remarquer que (i) le substrat β-napthyl-N-acétyl-β-D-glucosaminide [enzyme cible : N-acétyl-β-D-glucosaminidase] donne la même réaction positive avec notamment les souches isolées de ***Candida albicans***, ***Candida tropicalis***, ***Candida stellatoidea*** et ***Torulopsis glabrata***, et (ii) le substrat (6-bromo-β-naphtyl)-β-D-glucopyranoside [enzyme cible : β-D-glucosidase] donne la même réaction positive avec les souches isolées de ***Candida albicans***, ***Candida tropicalis***, ***Candida pseudotropicalis***, ***Torulopsis glabrata*** et ***Cryptococcus neoformans***. En d'autres termes, l'utilisation de ces substrats ne peut pas être directement appliquée à la détermination de ***Candida albicans*** dans un mélange contenant d'autres levures telles que les ***Candida*** et ***Torulopsis***.

En ce qui concerne l'utilisation de substrats monoaminoacides ou peptidiques sensibles aux amidases et notamment aux aminopeptidases dans le domaine de l'identification de souches de ***Candida*** et apparentées, on connaît deux solutions techniques.

La première solution représentée par le test commercialisé sous la dénomination YEAST-IDENT™ s'applique à des souches de levure préalablement isolées; la méthode d'identification repose sur la détermination en milieu liquide du profil enzymatique de la souche isolée, ledit profil étant obtenu au moyen d'un système d'une vingtaine de substrats différents après 4 h d'incubation à 37°C. Lesdits substrats, qui comportent un groupe fluorogène tel que β-naphtylamino (en abrégé : NA) sont notamment les H-Gly-NA, H-L-Pro-NA, H-L-Trp-NA, H-L-Hyp- NA, H-L-Ile-NA, H-L-Val-NA, H-L-Leu-Gly-NA, H-L-His-NA, H-L-Cys-NA, H-L-Tyr-NA et H-Gly-Gly-NA. Avec de tels substrats, l'interprétation est délicate. Comme ces substrats sont spécifiques d'enzymes appartenant à de nombreuses souches de levures et en particulier de ***Candida***, il faut tenir compte de l'ensemble de tous les résultats obtenus avec tous les substrats pour apprécier le profil enzymatique d'une souche isolée donnée de ***Candida***. L'utilisation de cette solution technique n'est pas directement transposable aux mélanges de souches, en effet le substrat particulier H-L-Pro-NA donne une réaction positive avec les souches isolées de ***Candida albicans***, ***Candida guillermondii***, ***Candida intermedia***, ***Candida parapsilosis***, ***Candida pseudotropicalis***, ***Candida stellatoidea***, ***Candida rugosa***, ***Candida zeylanoides*** et ***Torulopsis candida***.

La seconde solution décrite dans le résumé de R.W. KELLEY et al. paru dans Abstr. Annu. Meet. Am. Soc. Microbiol., C 337, page 384 (1986) et dans l'article de J.L. PERRY et al., J. Clin. Microbiol, 28 (No 3), pages 614-615 (mars 1990) concerne l'utilisation du substrat H-L-Pro-NA précité dans l'identification de ***Candida albicans*** et/ou ***Candida tropicalis***. Plus précisément, ledit résumé C 337 signale la possibilité de distinguer les souches de ***Candida albicans*** des souches de ***Candida tropicalis*** et propose à cet effet deux substrats, à savoir : le PNP-N-acétyl-β-D-galactosaminide et le H-L-Pro-NA. Cependant, ce résumé ne décrit la méthode qui permet de réaliser la distinction ***Candida albicans/ Candida tropicalis*** ni n'indique si ladite méthode s'applique à des mélanges de souches. L'article de J.L. PERRY et al. préconise l'utilisation simultanée de ces deux substrats dans une même réaction d'identification en phase liquide à partir de souches préalablement isolées sur milieu gélosé standard.

On sait de l'article de J.KUNERT et al., Journal of Medical and Veterinary Mycology 26, pages 187-194, (1988) que des substrats chromogènes comportant une chaîne monoaminoacide ou peptidique et dans lesquels le reste chromogène est le radical p-nitroanilino (en abrégé : pNA) ont été étudiés en vue de caractériser les enzymes protéolytiques extracellulaires de souches isolées de dermatophytes. Cet article cite notamment le substrat H-L-Pro-pNA mais ne concerne pas l'identification des ***Candida***, qui ne font pas partie de l'ensemble des dermatophytes, ni ne suggère l'utilisation dudit substrat pour la détermination de diverses souches de. ***Candida***, telles que celles de ***Candida albicans***, dans un mélange de ***Candida*** et de souches apparentées.

On sait par ailleurs que le brevet américain US-A-4 874 695 (date de publication : 17 octobre 1989) signale, d'une part, l'utilisation possible du substrat H-L-Pro-pNA dans l'identification de souches de levures (voir tableau I, colonne 4, ligne 36) et enseigne, d'autre part, que ce substrat n'est pas particulièrement spécifique dès lors qu'il est sensible vis-à-vis de plusieurs souches de ***Candida***, notamment les ***C. albicans***, ***C. guillermondii***, ***C. intermedia***, ***C. lipolytica***, ***C. lusitanica***, ***C. parapsilosis***, ***C. rugosa*** et ***C. zeylanoides*** (voir tableau IV, colonne 7, substrat No 8). Il convient de remarquer que selon ce brevet américain il n'est pas indiqué comment l'on peut rendre ce substrat spécifique des souches de ***Candida albicans***.

En conséquence, la lecture du brevet américain US-A-4 874 695 n'incite nullement l'homme de l'art à utiliser ledit substrat H-L-Pro-pNA pour l'identification de souches non-isolées de ***Candida albicans***.

### SOLUTION DE L'INVENTION

On propose suivant l'invention une nouvelle solution technique qui met en oeuvre des substrats chromogènes pour la détermination de souches de ***Candida albicans*** et qui est applicable à l'identification desdites souches parmi les plus usuelles, telles que les ***Candida tropicalis***, ***Candida krusei*** et ***Torulopsis glabrata*** (qui peuvent être pathogènes), d'une part, et les ***Candida guillermondii***, ***Candida pseudotropicalis***, ***Candida parapsilosis***, etc (qui sont peu pathogènes ou non pathogènes), d'autre part.

Plus précisément, cette nouvelle solution repose, dans l'identification de souches de ***Candida albicans*** dans un mélange de souches de ***Candida*** et de souches apparentées de l'ensemble des ***Torulopsis***, sur (i) l'utilisation d'un système de substrats chromogènes et (ii) l'utilisation d'un système de moyens inhibiteurs.

Cette solution technique offre l'avantage d'identifier parmi les ***Candida*** et ***Torulopsis***, qui comprennent principalement les levures pathogènes que l'on rencontre le plus souvent chez l'homme, les souches de ***Candida albicans***. A ce sujet, il est rappelé que les souches de ***Candida albicans***, de ***Candida tropicalis*** et de ***Torulopsis glabrata*** sont notamment responsables de vaginites, endocardites, pneumonies et septicémies, et que les souches de ***Candida krusei*** et ***Candida guillermondii*** peuvent être rencontrées le cas échéant chez l'homme.

### BUT DE L'INVENTION

Selon l'invention, on propose une nouvelle solution technique faisant appel à des substrats chromogènes et à un système de moyens inhibiteurs pour identifier dans un mélange de ***Candida*** et ***Torulopsis***, souches que l'on rencontre le plus souvent chez l'homme et qui sont notamment pathogènes, afin de distinguer principalement les ***Candida albicans*** des ***Torulopsis glabrata***, ***Candida tropicalis***, ***Candida krusei*** et ***Candida guillermondii***.

Ce procédé est directement applicable pour la détermination de souches non isolées de ***Candida albicans*** parmi d'autres souches, notamment les ***Torulopsis glabrata***, ***Candida tropicalis***, ***Candida krusei*** et ***Candida guillermondii***.

### OBJET DE L'INVENTION

Le but de l'invention et d'autres avantages sont obtenus par les caractéristiques données ci-après, pour l'identification des principales souches responsables des infections du type candidose que l'on rencontre en particulier chez l'homme.

On préconise un procédé pour l'identification de souches de ***Candida albicans*** parmi d'autres souches apparentées susceptibles d'être pathogènes et appartenant à l'ensemble des ***Candida*** et ***Torulopsis***, ledit procédé, qui met en oeuvre l'utilisation d'un substrat chromogène et d'un moyen inhibiteur, et qui n'implique pas l'isolation des souches à identifier, étant caractérisé en ce qu'il comprend les étapes selon lesquelles
(a) on met en contact un échantillon de souches à identifier avec un milieu nutritif comprenant (i) une source d'azote, une source de carbone et, le cas échéant, des oligo-éléments, (ii) un substrat chromogène sensible à au moins un enzyme appartenant à l'ensemble des aminoamidases et aminopeptidases et choisi parmi l'ensemble constitué par
   (α) H-L-Pro-pNA,
   (β) H-L-Hyp-pNA,
   (γ) H-Gly-L-Pro-L-Arg-pNA,
   (δ) Tos-Gly-L-Pro-L-Arg-pNA,
   (ε) H-L-4Hyp-L-Pro-L-Arg-pNA,
   (ζ) Boc-L-4Hyp-L-Pro-L-Arg-pNA, et
   (η) leurs sels d'addition,
   et (iii) un moyen inhibiteur constitué par un mélange gentamycine/cycloheximide; et,
(b) on incube puis observe pendant au moins 1 h la cinétique du clivage dudit substrat par la formation de couleur résultant dudit clivage.

### ABREVIATIONS

Dans la présente description, on a utilisé les abréviations suivantes par commodité :
- pour les restes d'aminoacide
   - Aib =: 2-aminoisobutyryle (ou 2-méthylalanyle)
   - Ala =: α-alanyle
   - Arg =: arginyle
   - ATC =: thiazolidine-4-carbonyle (ou thioprolyle)
   - Abu =: 2-aminobutyryle
   - CHA =: 3-cyclohexylalanyle
   - CHG =: α-cyclohexylqlycyle
   - CHT =: 3-(4-hydroxycyclohexyl)alanyle
   - Cys =: cystéyle
   - Gly =: glycyle
   - His =: histidyle
   - Hyp =: hydroxyprolyle (i.e. 3Hyp ou 4Hyp)
   - 3Hyp =: 3-hydroxyprolyle (ou 3-hydroxy-2-pyrrolidinecarbonyle)
   - 4Hyp =: 4-hydroxyprolyle (ou 4-hydroxy-2-pyrrolidinecarbonyle)
   - Ile =: isoleucyle
   - INC =: isonipécotyle [ou (pipéridin-4-yl)carnyle]
   - Leu =: leucyle
   - Lys =: lysyle
   - MeGly =: N-méthylglycyle (ou sarcosyle)
   - Nle =: norleucyle
   - Nva =: norvalyle
   - ONC =: 2-oxonipécotyle [ou (2-oxo-pipéridin-3-yl)carbonyle]
   - Orn =: ornithinyle
   - Phe =: phénylalanyle
   - Phg =: phénylglycyle
   - Pip =: pipécolinoyle
   - Pro =: prolyle
   - Ser =: séryle
   - Thr =: thréonyle
   - Trp =: tryptophanyle
   - Tyr =: tyrosyle
   - Val =: valyle
- pour les autres abréviations :
   - AcOH =: acide acétique
   - Adoc =: adamantyloxycarbonyle
   - Aoc =: t-amyloxycarbonyle
   - Boc =: t-butyloxycarbonyle
   - (6-Br)NA =: 6-bromo-β-naphtylamino
   - Bu =: n-butyle
   - Bz =: benzoyle
   - Cbo =: carbobenzoxy
   - Et =: éthyle
   - EM =: éthyloxymalonyle (ou EtO-CO-CH₂-CO)
   - Fmoc =: 9-fluorènylméthyloxycarbonyle
   - Foc =: furfuryloxycarbonyle
   - HTFA =: acide trifluoroacétique
   - Iboc =: isobornyloxycarbonyle
   - iPr =: isopropyle
   - Me =: méthyle
   - MeO =: méthoxy
   - (4-MeO)NA =: 4-méthyloxy-β-naphtylamino
   - MM =: méthyloxymalonyle (ou MeO-CO-CH₂-CO)
   - 4MU =: 4-méthylumbelliféryle
   - NA =: β-naphtylamino
   - ONP =: o-nitrophényle
   - PCB =: milieu de culture pomme de terre-carotte-bile
   - PEG =: polyéthylèneglycol
   - Ph =: phényle
   - pH =: cologarithme de la concentration en ions H⁺
   - PM =: poids moléculaire
   - pNA =: p-nitroanilino [ou (4-NO₂)C₆H₄NH]
   - PNP =: p-nitrophényle
   - Pr =: propyle
   - RAT =: milieu de culture riz-agar-tween
   - RT =: température ambiante (15-20°C)
   - tBu =: t-butyle
   - Tos =: p-toluènesulfonyle (ou tosyle)
   - TTC =: chlorure de 2,3,5-triphényl-2H-tétrazolium (ou rouge de tétrazolium)
   - YNB =: source d'azote commercialisée sous la nomenclature commerciale de "YEAST NITROGEN BASE"
   - Z =: benzyloxycarbonyle
   - Z (p-Cl) =: p-chlorobenzyloxycarbonyle
   - Z(p-OMe) =: p-méthoxybenzyloxycarbonyle.
- pour les notations des coloration et fluorescence :
   - - =: néant
   - + =: faible
   - ++ =: moyenne
   - +++ =: intense
   - ++++ =: très intense.

### DESCRIPTION DETAILLEE DE L'INVENTION

Les substrats chromogènes et leurs sels d'addition utiles suivant l'invention, qui présentent en général une plus grande sensibilité que les substrats fluorogènes (présence d'un groupe NA éventuellement substitué) vis-à-vis des souches de ***Candida*** et ***Torulopsis***, peuvent être représentés par la formule générale

R-(Aₙ)-A₁-R^{a} (Io)

dans laquelle
R représente l'atome d'hydrogène ou un groupe bloquant l'extrémité N-terminale,
Aₙ représente une simple liaison, un reste monoaminoacide ou un reste peptidique pouvant comporter de 2 à 5 restes d'aminoacide,
A₁ représente un reste α-aminoacide choisi parmi l'ensemble constitué par les restes Gly, MeGly, L-Arg, L-Lys, L-His, L-Pro, L-Hyp, L-Cys, L-Val et L-Trp; et,
R^{a} représente un reste chromogène aminé notamment choisi parmi l'ensemble comprenant le reste p-nitroanilino et les reste p-nitroanilino où le groupe phényle est substitué.

Les sels d'addition sont ici essentiellement des sels d'addition d'acide obtenus par réaction d'un composé de formule I avec un acide minéral ou organique, notamment HCl, HBr, AcOH ou HTFA.

Le groupe chromogène R^{a} qui convient d'une manière générale à la détermination quantitative des enzymes protéolytiques aminoamidases et aminopeptidases répond à la formule 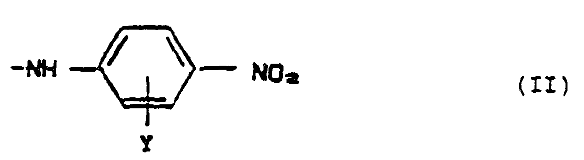 dans laquelle Y est H, Br, Cl, F, CF₃, COOH, COOW, CONH₂, CONHW, CONW₂, CONH(CH₂)ₘNMe₂, HOSO₂, CN, OH ou OW, où W est un groupe alkyle en C₁-C₆, aryle en C₆-C₁₀, aralkyle en C₇-C₁₁ ou alicyclique en C₃-C₈, et m est un nombre entier ayant pour valeur 1 à 10.

De tels groupes de formule II où le noyau phényle du groupe pNA est substitué sont notamment décrits dans le document EP-A-0 110 306. Parmi ces groupes on peut notamment citer les groupes utiles : pNA, 2-carboxy-4-nitroanilino et 3-carboxy-4-nitroanilino, 2-halogéno-4-nitroanilino et 3-halogéno-4-nitroanilino (où l'halogène est F, Cl ou Br), 2-méthoxy-5-méthyl-4-nitroanilino, 2-hydroxysulfonyl-4-nitroanilino, 4-trifluorométhyl-2-nitroanilino, 4-trifluorométhyl-3-nitroanilino, 4-cyano-2-nitroanilino, et analogues. Le groupe R^{a} préféré est pNA.

Le reste R représente H ou un groupe protecteur de l'extrémité N-terminale. Un tel groupe protecteur est bien connu dans la technique, par exemple un des restes bloquants l'extrémité N-terminale de peptides tels que décrits dans EP-A-0 110 306, US-A-4 480 030, FR-A-2 293 439 et US-A-4 440 678. Parmi les groupes R, qui sont différents de H, on peut notamment signaler les groupes alkyle en C₁-C₄ (notamment Me, Et, Pr, iPr, Bu, tBu), aryle éventuellement substitué (notamment Ph, tolyle, xylyle, chlorophényle, trifluorométhylphényle, méthoxyphényle), aralkyle éventuellent substitué (notamment Bzl, chlorobenzyle, dichlorobenzyle, trifluorométhylbenzyle, difluorobenzyle, méthoxybenzyle, éthoxybenzyle, 3,4-méthylènedioxybenzyle) et les groupes protecteurs classiques de l'extrémité N-terminale de peptides [notamment Ac, Tos, Adoc, Aoc, Bz, Cbo, Fmoc, Foc, Iboc, Z, Z(p-Cl) et Z(p-OMe)].

Les restes R sont ici choisis parmi l'ensemble constitué par (a) un reste oxymalonyle de formule R₁-O-CO-CH₂-CO, où R₁ est un groupe alkyle en C₁-C₄, un groupe phényle, un groupe phényle substitué par un ou plusieurs groupes Me, MeO, Cl, Br, F, CF₃, un groupe cycloalkyle en C₃-C₆, un groupe benzyle, un groupe benzyle substitué par un ou plusieurs groupes Me, MeO, Cl, Br, F, CF₃, ou un groupe cycloalkylméthyle où le fragment cycloalkyle est en C₃-C₆, et (b) un reste de polyléthylèneglycol de formule R₂-O(CH₂CH₂O)ₙ-CO, où R₂ est un groupe alkyle en C₁-C₆, phényle ou benzyle et n est un nombre entier ayant pour valeur 1 à 170.

D'une manière générale, on préfère ici les substrats où R est H car, à l'exception de quelques cas particuliers notamment pour les ***Candida guillermondii*** et ***Torulopsis glabrata***, la présence d'un groupe bloquant l'extrémité N-terminale diminue la cinétique du clivage par les aminoamidases ou les aminopeptidases.

Parmi les substrats chromogènes inclus dans la formule Io peuvent être mentionnés les substrats

R-A₁-R^{a} (I₁)

R-A₂-A₁-R^{a} (I₂)

R-A₃-A₂-A₁-R^{a} (I₃)

R-A₄-A₃-A₂-A₁-R^{a} (I₄)

et leurs sels d'addition
où R, A₁ et R^{a} sont définis comme indiqués ci-dessus et A₂, A₃, A₄ représentent chacun un reste aminoacide.

Ici, contrairement à la nomenclature classique des chaînes peptidiques, les restes d'aminoacide des substrats chromogènes sont numérotés 1, 2, 3, etc... à partir de l'extrémité CO-terminale et non à partir de l'extrémité N-terminale.

Quand Aₙ est un reste monoaminoacide (voir pour illustration la formule I₂), il représentera avantageusement Gly, L-Pro, L-Hyp, L-ONC, L-Phg ou L-ATC.

Quand Aₙ est un reste dipeptidique (voir pour illustration la formule I₃), le reste aminoacide en position 2 (i.e. A₂) sera avantageusement Gly, L-Pro, L-Hyp, ou L-Phg, et le reste aminoacide de l'extrémité N-terminale en position 3 (i.e. A₃) sera avantageusement Gly, D-Pro, L-Pro, D-Hyp, L-Hyp, D-Leu, L-Leu, D-Ala, L-Ala, D-Val, L-Val, D-ONC, L-ONC ou INC.

Quand Aₙ est un reste tripeptidique (voir pour illustration la formule I₄), le reste aminoacide en position 2 (i.e. A₂) sera avantageusement Gly, L-Pro, L-Hyp, L-Thr ou L-Phg, le reste aminoacide en position 3 (i.e. A₃) sera avantageusement Gly, L-Pro, L-Hyp, L-Leu, L-Ala, L-Ser ou L-Val, et le reste aminoacide de l'extrémité N-terminale (i.e. A₄) sera avantageusement Gly, D-Pro, L-Pro, D-Hyp, L-Hyp, D-Leu, L-Leu, D-Ala, L-Ala, D-Val, L-Val, D-ONC, L-ONC ou INC.

D'une façon avantageuse, Aₙ ne comportera suivant l'invention aucun reste CHA ou CHT.

Les substrats les plus intéressants suivant l'invention sont en général ceux dans lesquels Aₙ est une simple liaison, un reste monoaminoacide, dipeptidique ou encore tripeptidique.

De façon avantageuse A₁ sera L-Pro, L-Hyp, L-Arg ou L-Lys, et mieux L-Pro ou L-Arg.

En pratique, le reste aminoacide de l'extrémité CO-terminale du substrat chromogène sera toujours de configuration L; le reste aminoacide en position 2 sera un reste dépourvu d'atome de carbone asymétrique, tel que Gly, ou un reste α-aminoacide de configuration L; le reste aminoacide de l'extrémité N-terminale en position supérieure à 2 sera un reste dépourvu d'atome de carbone asymétrique, tel que Gly, ou un reste α-aminoacide de configuration D ou L; et, les éventuels restes aminoacides présents entre la position 2 et le reste aminoacide de l'extrémité N-terminale seront soit dépourvus d'atome de carbone asymétrique, soit de configuration L.

On a consigné dans les tableaux I à V ci-après un certain nombre de substrats chromogènes qui ont été testés vis-à-vis de plusieurs souches. Les résultats consignés dans les tableaux I-IV ont été obtenus à partir d'un milieu de culture tamponné à pH 6,0 comprenant une source d'azote (YNB; 6,7 g/l), du glucose (20 g/l), de l'agar (20 g/l), un antibiotique (gentamicine; 0,05 g/l) et le substrat à étudier. Le tableau I relatif à diverses souches de ***Candida*** fait également état d'essais comparatifs avec des substrats fluorogènes de l'art antérieur.

Ces tableaux montrent que, d'une manière générale, tous les substrats de formule I₁, I₂, I₃ et I₄, où R est H, sont hydrolysables par les aminoamidases et les aminopeptidases des souches de ***C.tropicalis***.

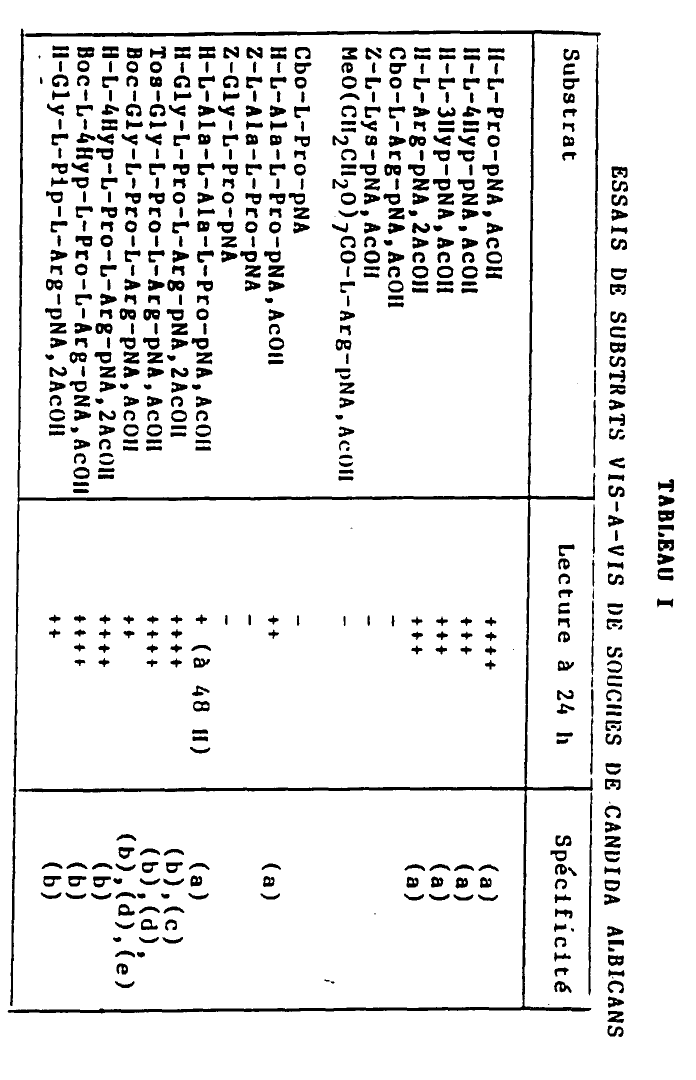

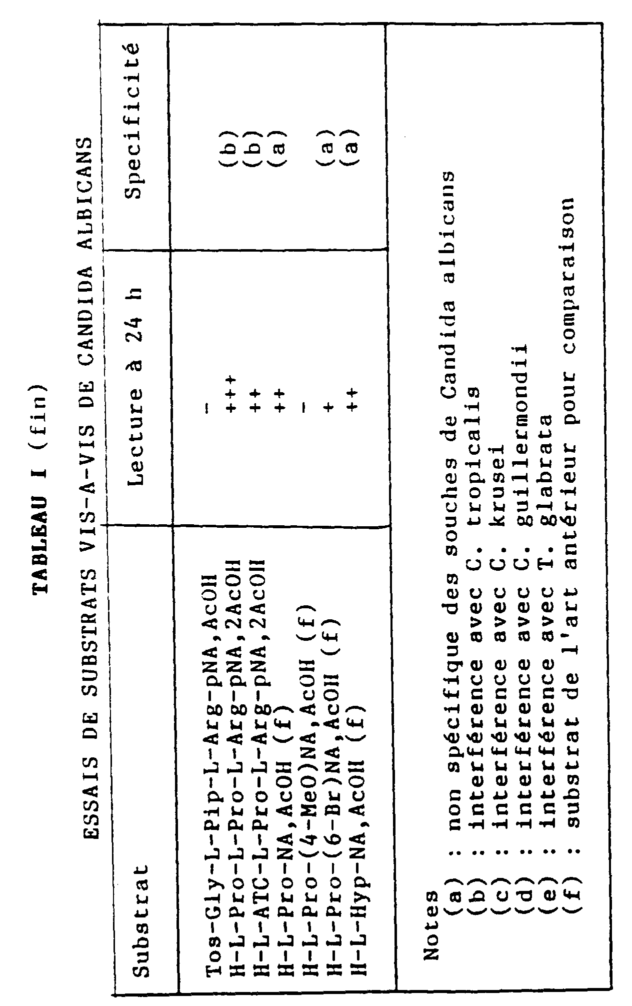

**TABLEAU II**

| ESSAIS DE SUBSTRATS VIS-A-VIS DE CANDIDA TROPICALIS | |
|---|---|
| Substrat | Lecture à 24 h |
| H-L-Pro-L-Arg-pNA,2AcOH | ++ |
| H-L-Pro-L-Pro-L-Arg-pNA,2AcOH | ++++ |
| H-D-Pro-L-Pro-L-Arg-pNA,2AcOH | ++++ |
| H-D-Pro-D-Pro-L-Arg-pNA,2AcOH | - |
| MM-L-Pro-L-Pro-L-Arg-pNA,AcOH | + |
| H-L-Hyp-L-Arg-pNA,2AcOH | ++ |
| H-L-Hyp-L-Lys-pNA,2AcOH | ++ |
| H-L-Hyp-L-Pro-L-Arg-pNA,2AcOH | +++ |
| H-L-Hyp-L-Hyp-L-Arg-pNA,2AcOH | +++ |
| H-D-Pro-L-Hyp-L-Arg-pNa,2AcOH | +++ |
| H₂NCO-L-Pro-D-Pro-L-Arg-pNA,AcOH | - |
| H₂NCO-D-Leu-Gly-L-Arg-pNA,AcOH | ++++ |
| H-L-Leu-Gly-L-Arg-pNA,2AcOH | ++++ |
| H-L-ONC-L-Pro-L-Arg-pNA,AcOH | +++ |
| H-INC-L-Pro-L-Arg-pNA,2AcOH | +++ |
| H-Gly-L-Pro-L-Arg-pNA,2AcOH | ++++ |

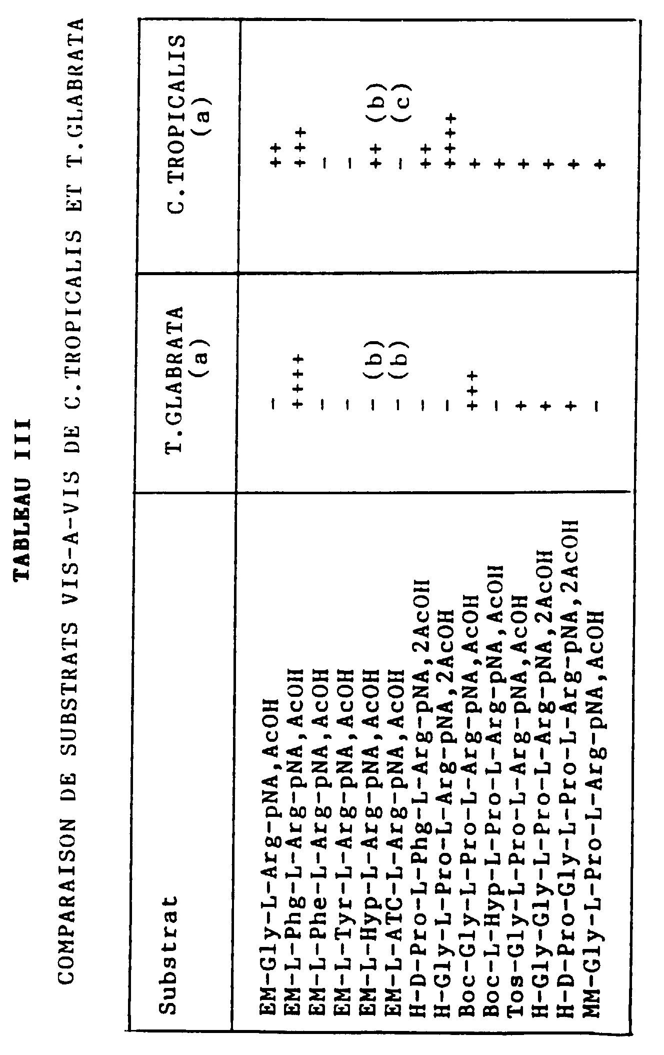

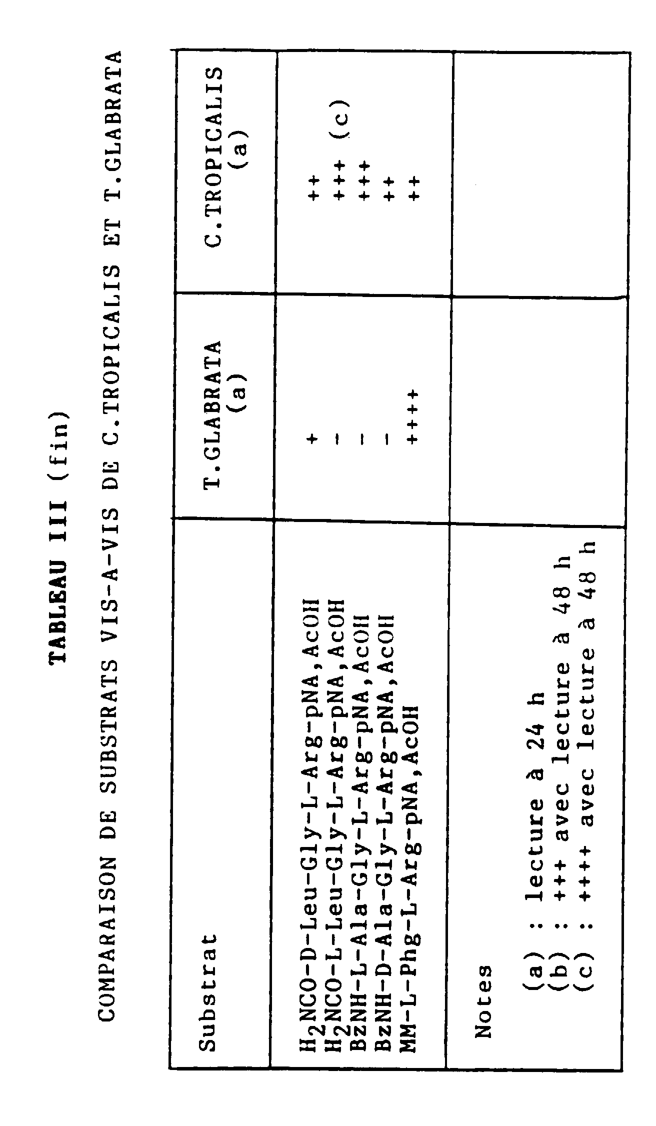

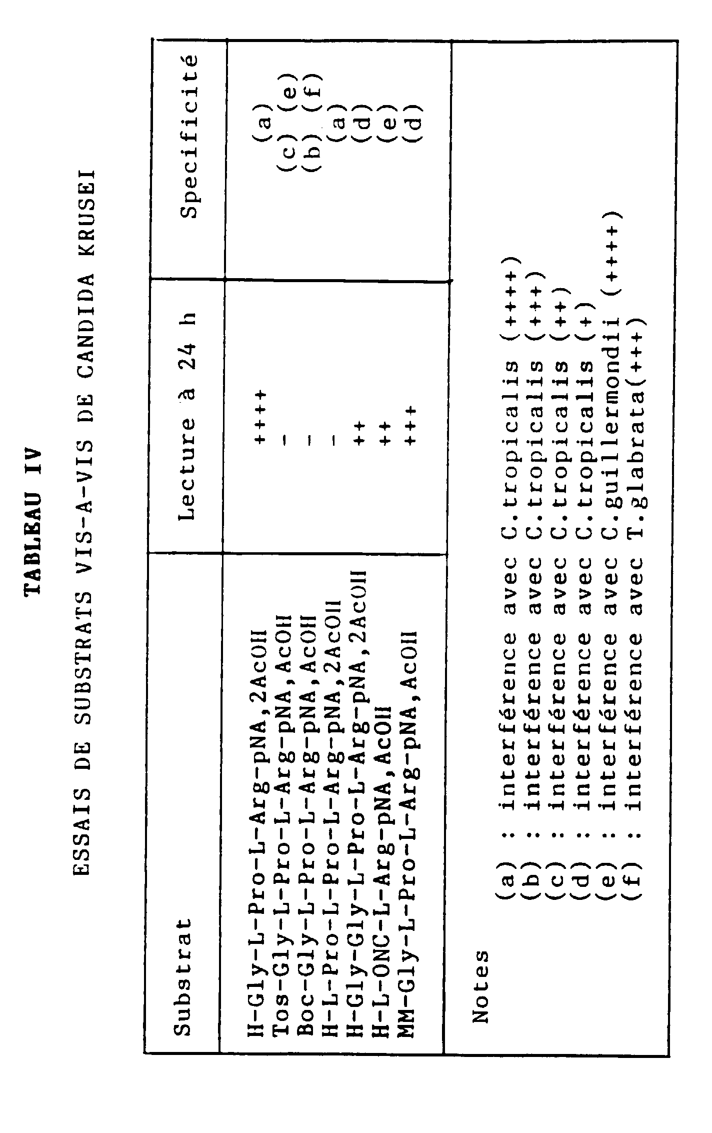

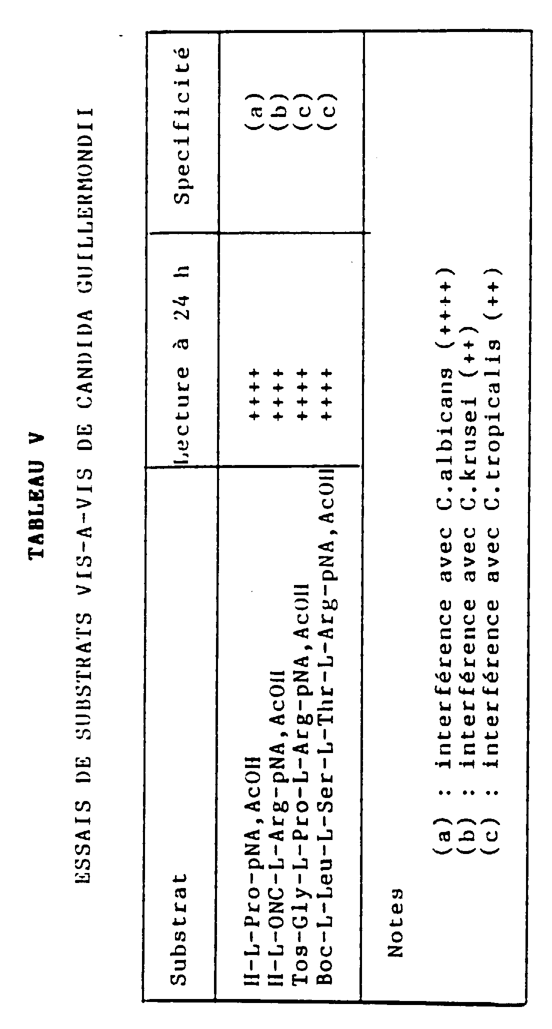

Les substrats chromogènes consignés dans les tableaux I-V ci-dessus et les tableaux donnés plus loin ont été préparés selon les méthodes classiques de la synthèse peptidique, notamment celles décrites dans l'art antérieur précité et la demande française n° 90 01965 du 19 février 1990 de la Demanderesse.

Le tableau I confirme que, pour identifier les souches de ***Candida albicans***, les substrats qui conviennent suivant l'invention sont choisis parmi l'ensemble constitué par les composés de formule

R-A₁-R^{a} (I₁)

où R, A₁ et R^{a} sont définis comme indiqué ci-dessus (notamment H-L-Pro-pNA et H-L-Hyp-pNA), et les composés de formules : 
H-Gly-L-Pro-L-Arg-pNA,
Tos-Gly-L-Pro-L-Arg-pNA,
H-L-4Hyp-L-Pro-L-Arg-pNA,
Boc-L-4Hyp-L-Pro-L-Arg-pNA,
 et leurs sels d'addition.

Il se trouve que les substrats de formule I₁, qui comportent comme aminoacide A₁ un reste L-Arg, L-Trp, L-Val, L-His ou L-Cys et qui sont hydrolysés par les souches de ***Candida albicans***, sont en général également hydrolysés par plus de 90 % des souches de ***T.glabrata***, ***C.krusei***, et ***C.tropicalis*** (qui sont susceptibles d'être pathogènes). En revanche, on a observé suivant l'invention que les substrats de formule I₁, où A₁ est L-Pro ou L-Hyp, et R est H, sont plus spécifiques des ***C.albicans*** dès lors qu'ils ne sont hydrolysés que par les ***C.guil*** ***lermondii***, ***C. parapsilosis***, ***C. zeylanoides*** et ***Tr. cutaneum*** (qui sont peu pathogènes ou non pathogènes).

On assure selon l'invention la spécificité du procédé d'identification en associant au substrat des inhibiteurs. En particulier, le cycloheximide incorporé au milieu de culture contenant le substrat de ***Candida albicans***, inhibe la pousse de toutes les levures appartenant à l'ensemble des ***Candida*** et des ***Torulopsis*** qui interfèrent sur la spécificité du substrat de ***Candida albicans***. Les quelques souches de ***Candida zeylanoides*** qui ne seraient pas inhibées par ledit cycloheximide peuvent être sélectivement inhibées en utilisant comme source de carbone le maltose, de préférence au glucose, pour assurer l'identification des ***Candida albicans***.

Pour la mise en oeuvre du procédé d'identification de l'invention, l'on ensemence un milieu nutritif contenant une source d'azote, une source de carbone et le cas échéant des oligo-éléments, d'une part, et un inhibiteur, d'autre part. De façon avantageuse, le milieu d'ensemencement sera un milieu solide (gélose), alors que le milieu d'ensemencement du brevet US-A-4 874 695 précité est liquide.

Comme inhibiteur, on recommande le mélange d'un antibactérien et d'un antifongique, à savoir : la gentamycine, d'une part, et le cycloheximide, d'autre part, qui, en présence d'un substrat spécifique de l'invention, tel que H-L-Pro-pNA, ne permettent que l'hydrolyse par les ***Candida albicans***, pour éliminer les contaminants ou sélectionner les souches que l'on veut identifier. En d'autres termes, la présence du cycloheximide inhibe la pousse des levures comprenant notamment les ***Torulopsis*** et les autres ***Candida*** interférant sur la détermination de ***Candida albicans***.

Le milieu de culture peut également comporter un colorant, notamment tel que la rhodamine (rhodamine 6G ou rhodamine B) à la concentration finale de 0,001-0,005 g/l, ou un colorant d'oxydo-réduction, notamment tel qu'un sel de tétrazolium [rouge de tétrazolium (TTC) ou encore bleu de tétrazolium] à la concentration de 0,05-0,9 g/l.

Les colorants interviennent en tant que moyens inhibiteurs de la culture de certains ***Candida***. En particulier, la rhodamine, et notamment la rhodamine 6G, inhibe la pousse de ***C.tropicalis*** et le sel de tétrazolium n'est pas réduit par les souches de ***T.glabrata*** et de ***C.krusei***.

La source d'azote sera avantageusement une peptone d'origine naturelle ou synthétique. Conviennent notamment, selon l'invention, les produits désignés sous les dénominations commerciales de "YEAST NITROGEN BASE" (YNB) référence 0392, NEOPEPTONE référence 0119, PROTEOSE PEPTONE référence 0122, BACTO PEPTONE référence 0118 (fournis par la société dite DIFCO) et l'extrait de levure référence 64341 (fourni par l'INSTITUT PASTEUR). La peptone sera utilisée à la concentration de 4 à 15 g/l, et de préférence à la concentration de 10 g/l.

La source de carbone sera constituée par un sucre, notamment le glucose ou le maltose, à la concentration de 10 à 40 g/l. Comme indiqué plus haut, le maltose qui permet la pousse des souches de ***Candida albicans*** offre l'avantage d'inhiber celle des souches de ***Candida zeylanoides***.

Le milieu de culture pourra être avantageusement solide et comportera à cet effet 10 à 30 g/l d'agar. Conviennent par exemple les produits commercialisés sous les nomenclatures de BACTO AGAR référence 0140, CORN MEAL AGAR référence 0386, BITEK AGAR référence 0138 (fournis par la société dite DIFCO).

Le milieu de culture pourra être tamponné à pH 4,5, à pH 5,0-5,5, et mieux à pH 6,0 (tampon acétate 0,1 M) ou à pH 8,0 (tampon Tris 0,1 M). Comme les souches de ***Candida guillermondii*** se cultivent à un pH supérieur ou égal à 6 et mieux en milieu alcalin, on peut éliminer l'éventuelle interférence de ***C.guillermondii*** sur la détermination de ***C.albicans*** en cultivant les souches de ***C.albicans*** isolées ou non-isolées à un pH de 4,5-5, dès lors que les souches de ***C.albicans*** sont en général résistantes dans les milieux acides de pH 4,5-5. Selon l'invention, il n'y a pas lieu de s'inquiéter de cette interférence quand on inhibe le développement des levures différentes de ***C.albicans*** au moyen de cycloheximide; il suffit alors d'opérer à pH 6,0 ou 8,0 en présence dudit cycloheximide.

Le meilleur mode de mise en oeuvre de l'invention consiste à :
(1) préparer un milieu de culture solide en incorporant dans 980 ml de tampon la peptone (10 g/l), le sucre (10 à 40 g/l), l'agar (10 à 30 g/l), homogénéiser le mélange résultant à RT, stériliser ledit mélange (notamment 0,25 h à 120-125°C), maintenir ledit mélange à 45-50°C après stérilisation pendant une durée inférieure à 4 h, ajouter une composition du substrat en milieu tampon préalablement stérilisée par passage sur membrane filtrante, la concentration du substrat étant comprise entre 0,5 et 2 mM et de préférence égale à 1 mM (soit par exemple une concentration finale de 0,35 g du substrat H-L-Pro-pNA par litre de gélose), ajouter la gentamycine (a) et le cycloheximide (d) et le cas échéant un autre des inhibiteurs (b, c ou e), préalablement stérilisés sur membrane selon les quantités :
   (a) solution tamponnée de gentamycine (à la concentration finale de 0,03-0,06 g, de préférence 0,05 g par litre de gélose),
   (b) solution tamponnée de sel de tétrazolium (de préférence TTC, à la concentration finale de 0,1 g par litre de gélose),
   (c) solution de chloramphénicol (à la concentration finale de 0,25 g par litre de gélose),
   (d) solution de cycloheximide (à la concentration finale de 0,1-0,5 g, de préférence 0,5 g par litre de gélose),
   (e) solution de rhodamine 6G (à la concentration finale de 0,001-0,003 g par litre de gélose),
   répartir le mélange résultant sous une forme convenable pour dosage (boîtes de Pétri, tubes, bandes ou autres), laisser refroidir ledit mélange conditionné pendant au moins 0,25 h à 20-25°C sous atmosphère stérile ;
   le produit ainsi obtenu est stocké à 12-15°C à l'abri de la lumière, il peut être placé dans des sacs ou poches appropriés, notamment plastiques pour empêcher toute évaporation ;
(2) ensemencer le milieu de culture ainsi conditionné avec une colonie de souche isolée ou d'un mélange de souches à étudier (l'ensemencement peut être réalisé soit par dépôt, soit par stries, soit encore par étalement), ladite colonie étant émulsifiée dans de l'eau distillée ou du sérum physiologique ;
(3) incuber le milieu de culture à une température comprise entre RT et 40°C (de préférence à 37°C) pendant au moins 18 h, à un pH supérieur ou égal à 4,5 et mieux compris entre 6,0 à 8,0;
(4) observer visuellement la réaction obtenue.

L'incubation aura en général une durée inférieure à 48 h. De préférence, ladite incubation sera réalisée à 37°C sur milieu solide gélosé pendant 20-30 h et mieux pendant 24 h.

La lecture du stade (3) permet d'apprécier la présence ou l'absence d'une réaction positive, la réaction positive se caractérisant notamment par :
- une coloration jaune visible à l'oeil nu due à la libération du reste chromogène préféré selon l'invention : H-pNA (ou coloration orangée due à la présence de la rhodamine), et
- un virage ou non du milieu en rose ou violet, selon l'espèce de ***Candida***, dû à la réduction du TTC.

D'autres avantages et caractéristiques de l'invention seront mieux compris à la lecture, qui va suivre, d'exemples de réalisation et d'essais comparatifs. L'ensemble de ces éléments n'est nullement limitatif mais est donné à titre d'illustration.

### EXEMPLE 1

Suivant le meilleur mode de mise en oeuvre précité, on prépare un milieu de culture renfermant les ingrédients suivants, dénommé gélose 1 ci-après, tamponné à pH 6,0

| | |
|---|---|
| YNB | 6,7 g/l |
| Glucose | 5,0 g/l |
| Agar | 20 g/l |
| Gentamicine | 0,04 g/l |
| Substrat chromogène | 1 mM |

### EXEMPLE 2

Suivant le meilleur mode de mise en oeuvre précité, on prépare un milieu de culture renfermant les ingrédients suivants, dénommé gélose 2 ci-après, tamponné à pH 6,0

| | |
|---|---|
| Sabouraud agar modifié (SM) | 50 g/l |
| Gentamicine | 0,05 g/l |
| Substrat chromogène | 1 mM |

### EXEMPLE 3

Suivant le meilleur mode de mise en oeuvre précité, on prépare un milieu de culture renfermant les ingrédients suivants, dénommé gélose 3 ci-après, tamponné à pH 6,0

| | |
|---|---|
| Sabouraud dextrose agar | 65 g/l |
| Gentamicine | 0,05 g/l |
| TTC | 0,1 g/l |
| Cycloheximide | 0,5 g/l |
| Substrat chromogène | 1 mM |

### EXEMPLE 4

Suivant le meilleur mode de mise en oeuvre précité, on prépare un milieu de culture renfermant les ingrédients suivants, dénommé gélose 4 ci-après, tamponné à pH 6,0

| | |
|---|---|
| YNB | 6,7 g/l |
| Glucose | 20 g/l |
| Agar | 20 g/l |
| Gentamicine | 0,05 g/l |
| Substrat chromogène | 1 mM |

### EXEMPLE 5

Suivant le meilleur mode de mise en oeuvre précité, on prépare un milieu de culture renfermant les ingrédients suivants, dénommé gélose 5, ci-après tamponné à pH 6,0

| | |
|---|---|
| YNB | 6,7 g/l |
| Maltose | 20 g/l |
| Agar | 20 g/l |
| Gentamicine | 0,05 g/l |
| Cycloheximide | 0,5 g/l |
| Substrat chromogène | 1 mM |

### EXEMPLE 6

On a procédé à l'ensencement et à l'incubation de plusieurs souches isolées de ***C.albicans***, ***C.tropicalis***, ***C.krusei***, ***C.guillermondii***, ***C.pseudotropicalis***, ***C.parapsilosis***, ***C.zeylanoides***, ***T.glabrata*** et ***Trichosporon cutaneum*** sur les milieux des exemples 1 à 5. On a constaté que, avec le substrat H-Pro-pNA,AcOH, la gélose 5 est la plus appropriée vis-à-vis des souches de ***Candida albicans***. Une partie des résultats obtenus a été consignée dans le tableau VI ci-après, où la colonne TTC fait état de résultats d'essais entrepris séparément sur un milieu gélosé standard contenant TTC. Ces résultats montrent l'intérêt de l'utilisation du cycloheximide pour rendre le substrat H-L-Pro-pNA,AcOH spécifique des souches de ***Candida albicans***.

### EXEMPLE 7

On a procédé à l'ensencement et à l'incubation de plusieurs souches isolées de ***C.albicans***, ***C.tropicalis***, ***C.krusei***, ***C.guillemondii*** et ***T.glabrata*** sur les milieux de l'exemple 2 [contenant soit le substrat de l'art antérieur N-Ac-β-D-galactosaminide-PNP, soit les substrats H-L-Pro-pNA,AcOH (spécifique de ***C.albicans**)*, EM-L-Phg-L-Arg-pNA,AcOH (spécifique de ***T.glabrata***) et H-Gly-L-Pro-L-Arg-pNA,2AcOH (spécifique de ***C.krusei***)] et de l'exemple 5 (contenant le substrat H-L-Pro-pNA, AcOH).

On a constaté que les substrats EM-L-Phg-L-Arg-pNA,AcOH et H-Gly-L-Pro-pNA,AcOH sont hydrolysés par les souches de ***C.tropicalis***. Les résultats obtenus sont consignés dans le tableau VII ci-après, où la colonne TTC fait état de résultats d'essais entrepris séparément sur un milieu gélosé standard contenant TTC.

**TABLEAU VI**

| DIAGNOSTIC DE CANDIDA ALBICANS | | | | | |
|---|---|---|---|---|---|
| (à partir de colonies isolées) | | | | | |
| Lecture à t = 24 h | | | | | |
| souches | n (a) | TTC | (1) | (2) | (3) |
| ***C.albicans*** | 10 | rose | ++++ | ++++ | ++++ |
| ***C.tropicalis*** | 4 | mauve | (d) | - | - |
| ***T.glabrata*** | 4 | (b) | - | - | - |
| ***C.krusei*** | 4 | (b) | - | - | - |
| ***C.guillermondii*** | 2 | (c) | - | +++ | - |
| ***C.zeylanoides*** | 1 | (c) | - | ++ | - |
| ***Tr.cutaneum*** | 1 | rose | ++ | + | - |
| ***C.parapsilosis*** | 5 | rose | - | +++ | - |
| ***C.pseudotropicalis*** | 2 | rose | - | - | - |
| Notes : | | | | | |

| | | | | | |
|---|---|---|---|---|---|
| (a) nombre de souches | | | | | |
| (b) blanc et faiblement rose dans un cas | | | | | |
| (c) blanc-rose | | | | | |
| (d) avec les 4 souches étudiées, 3 donnaient un résultat - et 1 un résultat +++ | | | | | |
| (1) gélose 2 contenant le substrat N-Ac-β-D-galactosaminide-PNP | | | | | |
| (2) gélose 2 contenant le substrat H-L-Pro-pNA,AcOH | | | | | |
| (3) gélose 5 contenant le substrat H-L-Pro-pNA,AcOH | | | | | |

**TABLEAU VII**

| DIAGNOSTIC DES PRINCIPAUX CANDIDA | | | | | | | |
|---|---|---|---|---|---|---|---|
| (à partir de colonies isolées) | | | | | | | |
| Lecture à t = 24 h | | | | | | | |
| souches | n (a) | TTC | (1) | (2) | (3) | (4) | (5) |
| ***C.albicans*** | 6 | rose | +++ | ++++ | - | - | ++++ |
| ***C.tropicalis*** | 9 | mauve | (c) | - | ++ | ++ | - |
| ***T.glabrata*** | 9 | (b) | - | - | +++ | - | - |
| ***C.krusei*** | 6 | blanc | - | - | - | +++ | - |
| Notes : | | | | | | | |

| | | | | | | | |
|---|---|---|---|---|---|---|---|
| (a) nombre de souches | | | | | | | |
| (b) blanc-rose | | | | | | | |
| (c) lecture positive (++) pour 1 seule des 9 souches testées négative pour les autres | | | | | | | |
| (1) gélose 2 contenant le substrat N-Ac-β-D-galactosaminide-PNP | | | | | | | |
| (2) gélose 2 contenant le substrat H-L-Pro-pNA,AcOH | | | | | | | |
| (3) gélose 2 contenant le substrat EM-L-Phg-L-Arg-pNA,AcOH spécifique de ***T.glabrata*** | | | | | | | |
| (4) gélose 2 contenant le substrat H-Gly-L-Pro-L-Arg-pNA,2AcOH spécifique de ***C.krusei*** | | | | | | | |
| (5) gélose 5 contenant le substrat H-L-Pro-pNA,AcOH et le cycloheximide | | | | | | | |

Les résultats du tableau VII montrent que l'on peut identifier en 24 h les quatre principales levures de ***Candida*** et de ***Torulopsis*** que l'on rencontre dans les cas pathologiques.

### EXEMPLE 8

On a procédé à l'ensencement et à l'incubation d'un mélange de plusieurs souches non-isolées de ***C.albicans***, ***C.tropicalis***, ***C.krusei***, ***C.guillemondii***, ***C.pseudotropicalis***, ***C. parapsilosis***, ***Cryptococcus neoformans***, ***Torulopsis glabrata*** et ***Trichosporon cutaneum*** sur les milieux de culture, selon les exemples 1 à 5, contenant le substrat de l'art anté-rieur : N-Ac-β-D-galactosaminide-PNP, ou le substrat selon l'invention : H-Pro-pNA,AcOH. Les résultats, qui ont été notamment obtenus avec les géloses 2 et 5, sont consignés dans le tableau VIII ci-après, où la colonne TTC fait état de résultats d'essais entrepris séparément sur un milieu gélosé standard contenant TTC.

### EXEMPLE 9

On a procédé à l'ensencement et à l'incubation d'un mélange de plusieurs souches non-isolées de ***C.albicans***, ***C.tropicalis***, ***C.krusei***, ***C.guillermondii***, ***C.pseudotropicalis***, ***C.parapsilosis***, ***C.zeylanoides***, ***C.ciferii***, ***C.cerevisiae***, ***Torulopsis glabrata*** et ***Trichosporon cutaneum*** sur les milieux de culture, selon l' exemple 5, contenant le substrat antérieur : N-Ac-β-D-galactosaminide-PNP, ou le substrat selon l'invention : H-Pro-pNA,AcOH. Les résultats, qui ont été obtenus avec la gélose 5, ont été consignés dans le tableau IX ci-après, qui met en évidence l'intérêt de l'utilisation de l'inhibiteur,dans le cas d'espèce le cycloheximide, en association avec le maltose, où la colonne TTC fait état de résultats d'essais entrepris séparément sur un milieu gélosé standard contenant TTC.

**TABLEAU VIII**

| DIAGNOSTIC DE CANDIDA ALBICANS | | | | | |
|---|---|---|---|---|---|
| (à partir de colonies non-isolées) | | | | | |
| Lecture à t = 24 h | | | | | |
| (nombre total de souches : 35) | | | | | |
| souches | n (a) | TTC | (1) | (2) | (3) |
| ***C.albicans*** | 13 | (b) | ++++ | ++++ | +++ |
| ***T.glabrata*** | 8 | blanc | - | - | - |
| ***C.krusei*** | 6 | (c) | - | - | - |
| ***C.tropicalis*** | 1 | - | - | - | - |
| ***C.pseudotropicalis*** | 1 | rose | - | - | - |
| ***C.parapsilosis*** | 2 | rose | - | +++ | - |
| ***Tr.cutaneum*** | 2 | (d) | (e) | ++ | - |
| ***Cr.neoformans*** | 1 | - | - | - | - |
| ***C.guillermondii*** | 1 | (b) | - | +++ | - |
| Notes : | | | | | |

| | | | | | |
|---|---|---|---|---|---|
| (a) nombre de souches | | | | | |
| (b) blanc-mauve | | | | | |
| (c) pas de culture, sauf dans un cas une culture faible | | | | | |
| (d) blanc-rose | | | | | |
| (e) résultat - pour 1 souche et résultat +++ pour l'autre souche | | | | | |
| (1) gélose 2 contenant le substrat N-Ac-β-D-galactosaminide-PNP | | | | | |
| (2) gélose 2 contenant le substrat H-L-Pro-pNA,AcOH | | | | | |
| (3) gélose 5 contenant le substrat H-L-Pro-pNA,AcOH | | | | | |

**TABLEAU IX**

| DIAGNOSTIC DE CANDIDA ALBICANS | | | | |
|---|---|---|---|---|
| (à partir de colonies non-isolées) | | | | |
| Lecture à t = 24 h | | | | |
| (nombre total de souches : 108) | | | | |
| souches | n (a) | TTC | (1) | (2) |
| ***C.albicans*** | 89 | (b) | ++++(c) | ++++ |
| ***T.glabrata*** | 8 | blanc | - | - |
| ***C.krusei*** | 1 | rose | - | - |
| ***C.tropicalis*** | 1 | mauve | - | - |
| ***C.pseudotropicalis*** | 2 | - | - | - |
| ***C.parapsilosis*** | 1 | rose | - | - |
| ***Tr.cutaneum*** | 2 | rose | - | - |
| ***C.zeylanoides*** | 1 | (d) | - | - |
| ***C.guillermondii*** | 1 | rose | - | - |
| ***C.ciferii*** | 1 | blanc | - | - |
| ***C.cerevisiae*** | 1 | - | - | - |
| Notes : | | | | |

| | | | | |
|---|---|---|---|---|
| (a) nombre de souches | | | | |
| (b) rose pour 87 souches, mauve pour 2 souches | | | | |
| (c) négatif pour 2 souches sur 89 | | | | |
| (d) rose-mauve | | | | |
| (1) gélose 5 contenant le substrat N-Ac-β-D-galactosaminide-PNP | | | | |
| (2) gélose 5 contenant le substrat H-L-Pro-pNA,AcOH | | | | |

## Revendications

1. Procédé pour l'identification de souches de ***Candida albicans*** parmi d'autres souches apparentées susceptibles d'être pathogènes et appartenant à l'ensemble des ***Candida*** et ***Torulopsis***, ledit procédé, qui met en oeuvre l'utilisation d'un substrat chromogène et d'un moyen inhibiteur, et qui n'implique pas l'isolation des souches à identifier, étant caractérisé en ce qu'il comprend les étapes selon lesquelles
(a) on met en contact un échantillon de souches à identifier avec un milieu nutritif comprenant (i) une source d'azote, une source de carbone et, le cas échéant, des oligoéléments, (ii) un substrat chromogène sensible à au moins un enzyme appartenant à l'ensemble des aminoamidases et aminopeptidases et choisi parmi l'ensemble constitué par
(α) H-L-Pro-pNA,
(β) H-L-Hyp-pNA,
(γ) H-Gly-L-Pro-L-Arg-pNA,
(δ) Tos-Gly-L-Pro-L-Arg-pNA,
(ε) H-L-4Hyp-L-Pro-L-Arg-pNA,
(ζ) Boc-L-4Hyp-L-Pro-L-Arg-pNA, et
(η) leurs sels d'addition,
et (iii) un moyen inhibiteur constitué par un mélange gentamycine/cycloheximide; et,
(b) on incube puis observe pendant au moins 1 h la cinétique du clivage dudit substrat par la formation de couleur résultant dudit clivage.

2. Procédé suivant la revendication 1, caractérisé en ce que l'on incube sur un milieu liquide à un pH supérieur ou égal à 4,5 et mieux compris entre 6,0 et 8,0 puis observe pendant au moins 1 h, la cinétique du clivage dudit substrat.

3. Procédé suivant la revendication 1, caractérisé en ce que l'on incube sur un milieu solide gélosé à un pH supérieur ou égal à 4,5 et mieux compris entre 6,0 et 8,0 puis observe, pendant au moins 12 h, la cinétique du clivage dudit substrat.

4. Procédé suivant l'une quelconque des revendications 1, 2 et 3, caractérisé en ce que ledit milieu de culture est solide et contient :
(1) de 4 à 15 g/l de peptone ;
(2) de 10 à 40 g/l de glucose ou de maltose ;
(3) de 10 à 30 g/l d'agar ;
(4) de 0,5 à 2 mM de substrat chromogène.

5. Procédé suivant la revendication 4, caractérisé en ce que ledit milieu de culture contient en outre :
(5) de 0,03 à 0,06 g/l de gentamycine, et
(6) de 0,1 à 0,5 g/l de cycloheximide.

6. Procédé suivant l'une quelconque des revendications 1 à 5, caractérisé en ce que l'incubation est réalisée à une température comprise entre 15°C et 40°C.

## Patentansprüche

1. Verfahren zur Identifizierung von *Candida albicans*-Stämmen unter anderen, verwandten Stämmen, die pathogen sein können und der Gruppe aus *Candida* und *Torulopsis* angehören, wobei das Verfahren, das ein chromogenes Substrat und einen Hemmstoff verwendet und das nicht die Isolation der zu identifizierenden Stämme beinhaltet, dadurch gekennzeichnet ist, daß es die Schritte umfaßt:
(a) In-Kontakt-bringen einer Probe von zu identifizierenden Stämmen mit einem Nährmedium, umfassend (i) eine Stickstoffquelle, eine Kohlenstoffquelle sowie gegebenenfalls Spurenelemente, (ii) ein chromogenes Substrat, das gegenüber mindestens einem Enzym aus der Gruppe, bestehend aus Aminoamidasen und Aminopeptidasen, empfindlich ist und ausgewählt ist aus der Gruppe, bestehend aus
(α) H-L-Pro-pNA,
(β) H-L-Hyp-pNA,
(γ) H-Gly-L-Pro-L-Arg-pNA,
(δ) Tos-Gly-L-Pro-L-Arg-pNA,
(ε) H-L-4Hyp-L-Pro-L-Arg-pNA,
(ζ) Boc-L-4Hyp-L-Pro-L-Arg-pNA sowie
(η) deren Additionssalzen,
und (iii) einen Hemmstoff, der aus einem Gentamycin/Cycloheximid-Gemisch besteht, und
(b) Inkubieren, gefolgt von Beobachten der Kinetik der Spaltung des Substrats während mindestens 1 Std. anhand der Farbentstehung, die durch die Spaltung hervorgerufen wird.

2. Verfahren nach Anspruch 1, gekennzeichnet durch Inkubation in einem wäßrigen Medium bei einem pH-Wert größer oder gleich 4,5 und stärker bevorzugt zwischen 6,0 und 8,0, gefolgt von Beobachten der Kinetik der Spaltung des Substrats während mindestens 1 Std..

3. Verfahren nach Anspruch 1, gekennzeichnet durch Inkubation in einem festen Gelmedium bei einem pH-Wert größer oder gleich 4,5 und stärker bevorzugt zwischen 6,0 und 8,0, gefolgt von Beobachten der Kinetik der Spaltung des Substrats während mindestens 12 Std..

4. Verfahren nach irgendeinem der Ansprüche 1, 2 und 3, dadurch gekennzeichnet, daß das Kulturmedium fest ist und enthält:
(1) 4 bis 15 g/l Pepton,
(2) 10 bis 40 g/l Glucose oder Maltose,
(3) 10 bis 30 g/l Agar,
(4) 0,5 bis 2 mM chromogenes Substrat.

5. Verfahren nach Anspruch 4, dadurch gekennzeichnet, daß das Kulturmedium außerdem enthält:
(5) 0,03 bis 0,06 g/l Gentamycin und
(6) 0,1 bis 0,5 g/l Cycloheximid.

6. Verfahren nach irgendeinem der Ansprüche 1 bis 5, dadurch gekennzeichnet, daß die Inkubation bei einer Temperatur, umfassend zwischen 15°C und 40°C, durchgeführt wird.

## Claims

1. A method for identifying strains of ***Candida albicans*** among other related strains susceptible to be pathogenic and belonging to the group of the ***Candida*** and ***Torulopsis***, said method, which involves the use of a chromogenic substrate and an inhibitor, and does not entail the isolation of the strains to be identified, comprising steps in which
(a) a sample of strains to be identified is brought into contact with a nutritient medium comprising (i) a nitrogen source, a carbon source and, if appropriate, oligo elements, (ii) a chromogenic substrate sensitive to at least one enzyme belonging to the group of the aminoamidases and aminopeptidases and selected from the group consisting of
(α) H-L-Pro-pNA,
(β) H-L-Hyp-pNA,
(γ) H-Gly-L-Pro-L-Arg-pNA,
(δ) Tos-Gly-L-Pro-L-Arg-pNA,
(ε) H-L-4Hyp-L-Pro-L-Arg-pNA,
(ζ) Boc-L-4Hyp-L-Pro-L-Arg-pNA, and
(η) their addition salts, and
(iii) an inhibitor which is composed of a gentamycin (cycloheximide mixture ; and,
(b) incubation is carried out and the kinetics of the cleavage of said substrate are observed for at least 1 h by the formation of color resulting from said cleavage.

2. A method according to claim 1 wherein incubation is carried out on a liquid medium at a pH greater than or equal to 4.5 and preferably of between 6.0 and 8.0 and the kinetics of the cleavage of said substrate are then observed for at least 1 h.

3. A method according to claim 1 wherein incubation is carried out on a solid gelose medium at a pH greater than or equal to 4.5 and preferably of between 6.0 and 8.0 and the kinetics of the cleavage of said substrate are then observed for at least 12 h.

4. A method according to any one of claims 1, 2 and 3 wherein said culture medium is solid and contains
(1) from 4 to 15 g/l of peptone ;
(2) from 10 to 40 g/l of glucose or maltose ;
(3) from 10 to 30 g/l of agar ; and
(4) from 0.5 to 2 mmol/l of chromogenic substrate.

5. A method according to claim 4 wherein said culture medium also contains
(5) from 0.03 to 0.06 g/l of gentamycin, and
(6) from 0.1 to 0.5 g/l of cycloheximide.

6. A method according to any one of claims 1-5 wherein the incubation is carried out at a temperature of between 15°C and 40°C.
